# EUROPÄISCHE PATENTSCHRIFT

(11) **EP 0 924 523 B1**
(45) Veröffentlichungstag und Bekanntmachung des Hinweises auf die Patenterteilung: **07.06.2006**
(21) Anmeldenummer: 98122888.5
(22) Anmeldetag: 02.12.1998
(51) Int. Cl.: G01N 33/86, G01N 33/483, G01N 33/49, C12Q 1/56

(54) **Verfahren und Diagnosemittel zum Nachweis von Hämostasestörungen**
Method and diagnostic means for the detection of haemostatic disorders
Méthode et moyens diagnostiques pour la détection des désordres hémostatiques

(30) Priorität: 19.12.1997 DE 19756773
(43) Veröffentlichungstag der Anmeldung: 23.06.1999
(73) Patentinhaber: Dade Behring Marburg GmbH, 35001 Marburg (DE)
(72) Erfinder: Kraus, Michael Dr., 35041 Marburg (DE); Schelp, Carsten Dr., 35041 Marburg (DE); Wiegand, Andreas, 34613 Schwalmstadt (DE)

(56) Entgegenhaltungen:
- WO-A-95/06877
- US-A- 3 996 345
- US-A- 5 567 596
- M. STEINER: "Changes in the distribution of platelet membrane proteins revealed by energy transfer" BIOCHIMICA ET BIOPHYSICA ACTA, Bd. 805, Nr. 1, 14. September 1984 (1984-09-14), Seiten 53-58, XP001038681
- BYSTRYAK S ET AL: "HOMOGENEOUS IMMUNOFLUORESCENCE ASSAY BASED ON DYE-SENSITIZED PHOTOBLEACHING" ANALYTICAL BIOCHEMISTRY, ACADEMIC PRESS, SAN DIEGO, CA, US, Bd. 225, 1995, Seiten 127-134, XP000882217 ISSN: 0003-2697

## Beschreibung

Die Erfindung betrifft Verfahren sowie Diagnosemittel zum Nachweis von Hämostasestörungen, dadurch gekennzeichnet, daß infolge einer Blutplättchenaggregation, einer Gerinnselbildung und/oder einer Gerinnselauflösung Substanzen in einen Abstand zueinander gebracht werden, der eine Wechselwirkung, insbesondere einen Energietransfer, zwischen den Substanzen erlaubt oder unterbindet und das Ausmaß an Wechselwirkung gemessen wird.

Der Blutstrom versorgt über das Gefäßsystem die Organe mit Nährstoffen und sorgt für die Entsorgung von Stoffwechselprodukten. Die Aufrechterhaltung eines offenen und doch gegen die Umwelt abgeschlossenen Gefäßsystems ist daher von vitaler Bedeutung. Durch das Zusammenspiel der gegenläufige Systeme der Blutgerinnung und der Fibrinolyse - die in einem als Hämostase bezeichneten Gleichgewicht zueinander stehen - wird dies ermöglicht. Eine Störung dieser Systeme manifestiert sich klinisch im Extremfall entweder in Thrombosen, d.h. ungewollten Verschlüssen des Gefäßsystems, oder in einer hämorrhagische Diathese, d.h. einer Blutung. Beides kann über Organversagen bis hin zum Tod führen. Diese sind eine der Haupttodesursachen in der westlichen Welt. Dem Nachweis einer erworbenen oder vererbten Hämostasestörung kommt daher eine wichtige Rolle in der klinischen Diagnose zu.

Die Prozesse der Hämostase beruhen auf dem Zusammenspiel von Blutgefäßen (Kontraktion, Dilatation), Zellen (dem gefäßständigen Endothel bzw. im Blutgefäß flottierenden Thrombozyten) und humoralen Faktoren. Entsprechend der physiologischen Reihenfolge der Prozesse wird zwischen primärer und sekundärer Gerinnung unterschieden. Bei der primären Gerinnung dominieren die zellulären Komponenten und sie findet den Abschluß in der Bildung von Thrombozytenaggregaten (primäres Gerinnsel). Bei der sekundären Gerinnung, die durch die zellulären Komponenten im Normalfall initiiert wird, dominieren die humoralen Faktoren. Bei diesen humoralen Faktoren handelt es sich um Proteine, die nach ihrer Funktion im wesentlichen in Enzyme, Cofaktoren und Stützproteine unterschieden werden. Bei der Aktivierung der Gerinnung werden über ein Kaskaden-ähnliches System Proenzmye durch aktive oder aktivierte Enzyme proteolytisch in ihre aktive Form umgesetzt. Diese Aktivität kann durch meist ebenfalls proteolyisch aktivierte Cofaktoren gesteigert werden. Als letzter Schritt erfolgt die Umsetzung des in seiner Vorform löslichen Stützproteins Fibrinogen durch das Gerinnungsenzym Thrombin in sein unlösliches Folgeprodukt Fibrin. Durch Aggregation und enzymatische Quervemetzung des Fibrins entsteht das Fbringerinnsel (sekundäres Gerinnsel). Primäre und sekundäre Gerinnung dienen der Wundheilung. Im pathologischen Fall jedoch kommt es zu einer ungewollten Verstopfung der Blutbahn, klinisch als Thrombose bezeichnet Die Auflösung der Gerinnsel erfolgt über das Fibrinolyse-System, das über eine ähnliche Aufeinanderfolge aktivierender Enzyme zur Aktivierung der Protease Plasmin führt. Im pathologischen Fall einer Hypefibrinolyse zerstört Plasmin unspezifisch noch nicht geronnenes Fibrinogen und stört dadurch die Integrität des kapillaren Systems, es kommt zu Blutungen.

Um etwaige Störungen der Hämostase zu erkennen, werden die oben beschriebenen Prozesse mit Hilfe von Diagnoseverfahren untersucht, die man in sogenannte
- klassische Verfahren,
- klinisch-chemische Verfahren sowie
- immunchemische Verfahren
unterscheidet.

Die als klassische Verfahren bezeichneten Diagnoseverfahren beruhen auf dem Nachweis einer Gerinnselbildung. Vorherrschend sind solche Verfahren, die die Aktivierungskaskaden der Gerinnung und/oder der Fibrinolyse an einer Stelle aktivieren und die Zeit bis zur Bildung bzw. Auflösung eines Gerinnsels messen.

Veränderungen dieser Gerinnungs- bzw. Fibrinolysezeiten gegenüber einer normalen Probe lassen Rückschlüsse auf pathologische Veränderungen des aktivierten Teilbereichs, dem Bereich der Reaktionskaskaden bis zur Bildung bzw. Auflösung eines Fibrinaggregats, zu. Üblicherweise erfolgt die Detektion des Gerinnsels bei den klassischen Verfahren mittels mechanischer oder optischer Nachweisverfahren.

Bei mechanischer Detektion wird die erhöhte Viskosität der gerinnenden Probe oder die Bildung von Fibrinfäden genutzt. So wird beispielsweise eine Kugel oder eine Rührstab in den Boden eines Reaktionsgefäßes eingesetzt und die Gerinnung in der dazugegebenen Blutprobe ausgelöst. Die Kugel bzw. der Rührstab oder das Gefäß rotieren und die Kraft für diese Bewegung wird gemessen. Erhöht sich auf Grund der Plättchenaggregation oder der Gerinnselbildung die Viskosität der Lösung wird dies als erhöhter Widerstand gemessen und ab einem bestimmten Wert als Eintritt der Gerinnung gewertet. Bei einem anderen Verfahren (nach Schnitger & Gross) taucht eine hakenförmige Elektrode in die Probe in rhytmischen Abständen ein und aus. Dabei wird entsprechend die Stromzufuhr zu einer in der Probe liegenden Elektrode ein- und ausgeschaltet. Bildet sich ein Fibrinfaden, so verfängt sich die bewegliche Elektrode und der Stromfluß bleibt erhalten. Dies wird als Eintritt der Gerinnung interpretiert. Bei der Fibrinolysediagnostik wird umgekehrt die Auflösung des Gerinnsels durch eine Verringerung der Viskosität bzw. die Auflösung des Fibrins entsprechend detektiert.

Die mechanische Detektion bei den klassischen Verfahren hat den Vorteil, daß sie die physiologisch wichtige Eigenschaft der Gerinnselbildung, nämlich den mechanisch stabilen Verschluß einer Wunde, erfaßt. Demgegenüber erfordert diese Technik spezielle Geräte, die nur für diese speziellen Hämostase-Teste geeignet sind.

Unter optischer Detektion wird die turbidimetrische Bestimmung der Trübungsänderung der Lösung bei Entstehung eines Gerinnsels verstanden. Das Trübungssignal bei einem Fibringerinsel kann durch dem Fachmann bekannte Verfahren verstärkt werden, wie etwa durch Erhöhung der optischen Dichte der Probe, beispielsweise durch Zugabe von Partikeln bzw. durch eine Verstärkung der Denaturierung des Fibrins durch Zugabe von Salzen bzw. Ionen, beispielsweise von Metallionen wie Mangan-, Eisen- oder Calciumionen. Unter optischer Detektion bei den klassischen Verfahren wird nicht die ebenfalls photooptische meßbare Umsetzung chromogener Substrate verstanden, da damit eine Enzymaktivität, nicht aber die Aggregation von Thrombozyten oder die Umsetzung des natürlichen Stützproteins Fibrinogen erfaßt wird.

In EP 0 039 885 ist beschrieben, daß hydrophobe Latexpartikel zum Nachweis von Fibrinmonomeren, d.h. noch nicht polymerisierten Fibrins, verwendet werden können. Solche einfachen Latexpartikel sind jedoch nicht zum Nachweis eines Gerinnsels geeignet, da die Agglutination von Latexpartikeln eine erhöhte Lichtstreuung verursacht, die von der Lichtstreuung, die ein Fibringerinnsel selbst erzeugt, nicht unterschieden werden kann.

In der US 5 567 596 wird ein Verfahren zum Nachweis einer Proteinpolymerbildung bzw. -abbaus beschrieben, das dadurch gekennzeichnet ist, dass das für den Polymerisierungsprozess genutzte Proteinsubstrat durch ein Fluoreszenzlabel markiert ist und dass dieses Substrat ein "Template" des Proteins darstellt, welches in das Proteinpolymer eingebaut wird. Der Nachweis erfolgt über ein Quenching der Lichtemission des Labels.

Im Verfahren gemäß Steiner et al., *Biochim.Biophys.Acta* 805 (1984), Seiten 53 - 58 werden mittels Antikörper oder Lektine Fluoreszenzlabel an Proteine in Blutplättchenmembranen spezifisch gebunden. Dieses Verfahren dient zur Untersuchung der Proteinverteilung innerhalb der Membran nach Blutplättchenaktivierung.

Ein Vorteil der optische Detektion ist die Möglichkeit, die Messungen auf gängigen klinisch-chemischen Analyseautomaten durchzuführen. Da die optische Detektion aber auf einer Trübungsmessung beruht, können im Gegensatz zur mechanischen Detektion Trübungen der Proben, z.B. bei hyperlipämischen Proben, die Bestimmung stören. Im Extremfall, wie beispielsweise in Vollblut, ist auf Grund der optischen Dichte gar keine optische Detektion möglich. Dies ist ein wesentlicher Nachteil dieser optischen Methoden.

Neben den oben beschriebenen gebräuchlichen klassischen Diagnoseverfahren sind in der Hämostasediagnostik auch die klinisch-chemischen Verfahren gebräuchlich. Hierbei wird über den Umsatz spezifischer, chromogener Substrate allein oder in Kombinationen mit Enzymen und/oder Zwischenprodukten (enzymatisch gekoppelter Test) die Aktivität einzelner Enzyme gemessen. Diese Bestimmungen sind unabhängig von der Bildung eines Thrombozytenaggregats oder eines Fibringerinnsels. Nachteilig ist hierbei jedoch das die physiologisch relevante Reaktionen, nämlich Störungen bei der Bildung eines Thrombozytenaggregats oder eines Fibringerinnsels bzw. deren Auflösung, z.B. eine Dysfibrinogenämie oder eine eingeschränkte Reaktivität der Gerinnungsenzyme mit Phospholipidoberflächen aufgrund eines Vitamin K-Mangels bzw. einer Coumarin-Therapie, nicht erfaßt werden.

Die immunchemischen Verfahren sind in der Hämostasediagnostik eher unüblich, da hier die Diagnose funktioneller Störungen und weniger die Bestimmung der Menge eines nachzuweisenden Analyten im Vordergrund stehen. Allerdings läßt der immunchemische Nachweis von Spaltprodukten aus der proteolytischen Wirkung der Kaskadenreaktionen oder der Nachweis von Protease/Inhibitor-Komplexen Rückschlüsse auf die aktuelle Aktivität der jeweiligen Systeme zu. Diese können für die Differentialdiagnostik hilfreich sein. Derzeit beschränkt sich die Anwendung der immunchemischen Methoden in der Hämostasediagnostik vorwiegend auf Forschungsarbeiten.

Der Erfindung lag die Aufgabe zugrunde eine neue Detektionsmethode zu finden, die es ermöglicht, auch die klassischen mechanischen Verfahren auf gängigen Analyseautomaten durchzuführen und die sich als robust gegenüber den bei den optischen Verfahren üblichen Störungen durch Probentrübungen erweist, so daß auch eine Messung im Vollblut ermöglicht wird.

Diese Aufgabe wird erfindungsgemäß gelöst durch ein Verfahren bzw. durch die Verwendung eines Diagnosemittels gemäß einem oder mehreren der Ansprüche 1-25, insbesondere durch ein Verfahren zum Nachweis einer Hämostasestörung, bei den infolge einer Blutplättchenaggregation, einer Gerinnselbildung und/oder einer Gerinnselauflösung Substanzen in einen Abstand zueinander gebracht werden, der eine Wechselwirkung, insbesondere einen Energietransfer, zwischen den Substanzen erlaubt oder unterbindet und das Ausmaß an Wechselwirkung gemessen wird, dadurch gekennzeichnet, dass eine oder mehrere dieser Substanzen an suspendierbare Partikel kovalent, über spezifische Wechselwirkung und/oder adsorptiv gebunden sind und/oder in diese eingelagert sind oder selbst suspendierbare Partikel darstellen oder ein Teil derselben sind.

Unter dem Begriff einer Hämostasestörung sind alle vererbten oder erworbenen Störungen des Gerinnungs-, Fibrinolyse- und auch des Komplementssystems gemeint, insbesondere Mangel oder Defizite der daran beteiligten Faktoren und Regulatoren, sowie von Rezeptoren für diese, soweit diese durch die Präsenz von Gewebe, Gewebeteilen und/oder Zellen im Testansatz zum Einsatz kommen.

Durch die Bestimmung des Ausmaßes an Wechselwirkung zwischen den Substanzen - z.B. durch die direkte oder indirekte Messung der übertragenen Energiemenge - kann auf den Prozeß der Blutplättchenaggregation, der Gerinnselbildung und/oder der Gerinnselauflösung rückgeschlossen werden. Mit diesem Verfahren lassen sich daher die Vorteile der klassischen mechanischen (direkte Messung des physiologischen Vorganges der Gerinnselbildung bzw. - auflösung) und der klassischen optischen Hämostase-Diagnoseverfahren (Messung an üblichen Analyseautomaten) verbinden, ohne dabei deren Nachteile (z.B. Störempfindlichkeit gegenüber trüben Proben) zu übernehmen. So können mit dem erfindungsgemäßen Verfahren demnach alle in der Hämostase-Diagnostik üblichen Proben wie z.B. Blutplättchen-armes Plasma, Blutplättchen-reiches Plasma oder Vollblut gemessen werden.

Vollblut stellt das Maximum einer Störung der optischen Verfahren dar. Auf Grund der anwesenden Erythrozyten ist die optische Dichte so hoch, daß an gängigen optisch messenden Geräten nicht gemessen werden kann. Der mit dem erfindungsgemäßen Verfahren ermittelte Reaktionsverlauf der Gerinnung von Vollblutproben ist praktisch unbeeinflußt. Dies ermöglicht die Anwendung des erfindungsgemäßen Verfahrens auf die Bestimmung von Vollblutproben in der Patienten-nahen (Point of Care) oder Heim-Diagnostik. Da bei der Vollblutgerinnung primäre und sekundäre Gerinnung zusammenspielen, kann der Gesamtvorgang mit dem erfindungsgemäßen Verfahren bestimmt werden. Das erfindungsgemäße Verfahren ist demnach sowohl zur Bestimmung der humoralen Faktoren als auch zur Bestimmung der Gerinnungs- bzw. Lyseaktivität der zellulären Komponenten zu verwenden.

Das erfindungsgemäße Verfahren erlaubt die Durchführung der gesamten Hämostasediagnostik an einem einzigen Gerät, denn auch die entsprechenden klinisch-chemischen sowie immunchemischen Hämostase-Diagnoseverfahren können auf das erfindungsgemäße Verfahren hin adaptiert werden: Anstatt der üblichen chromogenen Substrate können beispielsweise zu einem effektiven Energietransfer befähigte Substanzen, die z.B. über eine entsprechende enzymatisch spaltbare Peptidkette verknüpft sind, eingesetzt werden (siehe z.B. WO 97/28261). Eine oder mehrere der Substanzen können dabei direkt oder indirekt z.B. über Verknüpfungssysteme wie Antigen/Antikörper oder Avidin/Biotin mit der Peptidkette verknüpft sein, so daß die enzymatische Reaktion vor oder nach der Verknüpfung der Substanz(en) an die Peptidkette oder auch zeitgleich zur Verknüpfung stattfinden kann.

Unter dem Begriff "Substanzen" sind im Rahmen der Gesamterfindung Mitglieder biologischer und/oder chemischer Stoffklassen zu verstehen, die bei räumlicher Nähe miteinander in Wechselwirkung treten können, z.B. in Form von Energiespendem und Energieempfängem wie beispielsweise Photosensitizer und Chemiluminescer (EP 0 515 194; Ullman et al. (1996) Clinical Chemistry 42:1518 - 1526), Photosensitizer und Fluorophore (WO 95/06877; Bystrak et al. (1995) Anal. Biochem. 225:127 - 134), oder radioaktives lod¹²⁵ und Fluorophore (S. Udenfriend et al. (1985) Proc. Natl. Acad. Sci. 82:8672 - 8676), oder Fluorophore und Fluorophore (Mathis,G. (1993) Clin. Chem. 39:1953 - 1959) oder Fluorophore und Fluoreszenz-Quencher (US 3,996,345).

Unter einer Wechselwirkung zwischen den Substanzen ist insbesondere ein Energietransfer - also die direkte Übertragung von Energie zwischen den Substanzen, z.B. durch Licht- oder Elektronenstrahlung sowie über reaktive chemische Moleküle - zu verstehen. Der Energietransfer kann von einer auf eine andere Substanz erfolgen, möglich ist aber auch eine Kaskade verschiedener Substanzen über die der Energietransfer läuft.

Femer umfaßt sind vom Begriff "Wechselwirkung zwischen den Substanzen" auch Vorgänge, bei denen die Aktivität einer Substanz durch eine oder mehrere andere inhibiert oder verstärkt wird, beispielsweise die Hemmung oder Steigerung der Enzymaktivität oder die Hemmung, Steigerung oder Veränderung (z.B. Wellenlängenverschiebung, Polarisation) des von der beeinflußten Substanz ausgesendeten Lichtes.

Desweiteren sind unter dem Begriff einer Wechselwirkung zwischen den Substanzen auch Enzymkaskaden zu verstehen (s.a. US 4,663,278). In diesem Fall sind die Substanzen Enzyme, von denen mindestens eines das Substrat für ein anderes liefert, dadurch gekennzeichnet, daß infolge einer Blutplättchenaggregation, einer Gerinnselbildung und/oder einer Gerinnselauflösung die Enzyme in einen Abstand zueinander gebracht werden, so daß eine maximale oder minimale Reakionsgeschwindigkeit der gekoppelten Substratumsetzung resultiert. So lassen sich beispielsweise folgende Varianten realisieren:
(a) Enzym E1 (= Substanz A) erzeugt das Substrat "a" für das Enzym E2 (= Substanz B), welches vom Enzym E2 zu einem meßbaren End- oder Zwischenprodukt "b" umgesetzt wird. Sind die Enzyme in Lösung, so ist der Diffusionsweg für das Substrat "a" zum Enzym E2 relativ weit und damit die Umsetzungskinetik zu "b" langsam. Liegen die Enzyme E1 und E2 z.B. infolge einer Gerinnselbildung oder Blutplättchenaggregation räumlich benachbart vor, dann ergibt sich eine schnellere Umsetzungskinetik zu "b".
(b) Die Enzyme E1 (=Substanz A) und E3 (=Substanz C) liegen von Anfang an in räumlicher Nähe vor; sie sind z.B. beide an die gleichen Teilchen gebunden. Das Enzym E1 bildet das Substrat für das Enzym E2 (Substanz B), wobei das Enzym E2 für das Enzym E3 das Substrat erzeugt. Das Verfahren erfolgt in Analogie zu (a), wobei infolge einer Gerinnselbildung oder Blutplättchenaggregation alle drei Substanzen in räumliche Nähe gebracht werden oder infolge einer Gerinnselauflösung eine in räumliche Nähe vorliegende Anordnung aufgehoben wird.

Eine effektive Wechselwirkung zwischen den Substanzen findet statt, wenn diese räumlich benachbart vorliegen, also z.B. innerhalb eines Abstandbereiches von wenigen µm, insbesondere innerhalb eines Abstandbereiches von unter 600 nm, bevorzugt unter 400 nm, ganz besonders bevorzugt von unter 200 nm.

In einer bevorzugten Ausführungsform des erfindungsgemäßen Verfahrens erfolgt die Wechselwirkung zwischen den Substanzen als Energietransfer z.B. mittels
- kurzlebiger Moleküle, z.B. Singulett-Sauerstoff (s.a. EP 0 515 194; Ullman et al. (1994) Proc. Natl. Acad. Sci. 91:5426-5430; Ullman et al. (1996) Clinical Chemistry 42:1518 - 1526, WO 95/06877 und Bystrak et al. (1995) Anal. Biochem. 225: 127 - 134),
- Strahlung geringer Reichweite, z.B. radioaktive β-Strahlung (s. Hart & Greenwald (1979) Molecular lmmunology 16:265-267 und Udenfriend et al. (1985) Proc. Natl. Acad. Sci. 82:8672-8676),
- und/oder Energietransfers nach Förster (Mathis, G. (1993) Clin. Chem. 39:1953-1959; US 5,527,684).

Eine weitere bevorzugte Ausführungsform des erfindungsgemäßen Verfahrens ist dadurch gekennzeichnet, daß die Aktivität von Substanzen durch andere Substanzen verstärkt oder inhibiert wird und dies zu einer meßbaren Signaländerung führt wie beispielsweise die Änderung der Intensität oder der Polarisation des ausgestrahlten Lichtes, die Inhibition oder Steigerung von Enzymaktivitäten und/oder die Änderung des Fluoreszenzverhaltens.

Umfaßt vom erfindungsgemäßen Verfahren sind auch Ausführungsformen bei denen mindestens eine der Substanzen unspezifisch, z.B. durch hydrophobe oder elektrostatische Wechselwirkung, an Komponenten eines sich bildenden oder auflösenden Blutgerinnsels binden kann. Diese Bindung mag direkt über eine Wechselwirkung von Substanz und den Komponenten des sich bildenden oder auflösenden Blutgerinnsels erfolgen oder indirekt, indem die Substanz über bindungsvermittelnde Komponenten - wie beispielsweise mit der Substanz verbundene natürlich vorkommende oder synthetisch hergestellte Polymere, Proteine, insbesondere Fragmente des Fibrinogens, Zucker, Lipide oder Peptide, insbesondere solche, die die Aminosäuresequenz RGD aufweisen, oder besonders bevorzugt Partikel - an sich bildende oder auflösende Blutgerinnsel gebunden wird.

Die Erfindung ist dadurch gekennzeichnet, daß eine oder mehrere Substanzen an suspendierbare Partikel kovalent, über spezifische Wechselwirkung und/oder adsorptiv gebunden sind und/oder in diese eingelagert sind oder selbst suspendierbare Partikel darstellen oder ein Teil derselben sind. Unter dem Begriff suspendierbare Partikel sind Teilchen zu verstehen wie z.B. Farbstoffkristalle, Metallsolen, Silica-Partikel, Magnetpartikel, Öltropfen, Lipidpartikel, Dextran, Proteinaggregate oder besonders bevorzugt Latexpartikel. Bevorzugt werden Partikel mit einem Durchmesser von 0,01 - 10 µm, besonders bevorzugt mit einem Durchmesser von 0,05 - 3 µm, ganz besonders bevorzugt mit einem Durchmesser von 0,05 - 1 µm.

Bei einer kovalenten Bindung sind die Substanzen über eine chemische Bindung mit den Teilchen oder etwaigen, die Teilchen umhüllenden Schalen oder Schichten verbunden. Substanzen können auch über spezifische Wechselwirkungen mit den Teilchen oder Teilchenumhüllungen - z.B. vermittelt über Antikörper, Lektine, Rezeptoren, Biotin/Avidin, komplementären Nukleotidsträngen - verbunden sein. Die adsorptive Bindung ist im Regelfall auf hydrophobe, hydrophile oder elektrostatische Wechselwirkungen zwischen den Teilchen oder Teilchenumhüllungen und den Substanzen zurückzuführen. Effektiv ist auch eine Einlagerung der Substanzen in einen oder mehrere Teilchenhohlräume, die auch von Teilchenumhüllungen gebildet sein können.

Vom erfindungsgemäßen Verfahren eingeschlossen sind auch Ausführungsformen bei denen die Oberfläche der Partikel nach deren Herstellung weiter modifiziert wird und/oder die Partikel von einer oder mehreren kovalent oder adsorbtiv gebundenen Schichten oder Schalen - z.B. aus Proteinen, Kohlehydraten, lipophilen Substanzen, Biopolymeren, organischen Polymeren oder Mischungen hiervon - umhüllt sind, um z.B. Verbesserungen zu erreichen hinsichtlich Suspensionsstabilität, Lagerstabilität, formgebende Stabilität oder Resistenz gegen UV-Licht, Mikroben oder sonstige zerstörend wirkende Agenzien. Ebenfalls können die Modifikationen u. Umhüllungen dazu dienen die unspezifische Bindung an Oberflächen von Reaktionsgefäßen sowie an die von Probenbestandteilen wie insbesondere Proteinen (z.B. Albumin oder Antikörper) oder Zellbestandteilen (z.B. Phospholipiden oder Nukleinsäuren) zu reduzieren oder zu unterbinden. Weiterhin können die Modifikationen u. Umhüllungen dazu dienen die Hydrophobizität der Partikeloberfläche oder die Ladung der Oberfläche der Partikel zu erhöhen oder zu erniedrigen.

Durch derartige Veränderungen der Partikeloberfläche ist eine verbesserte selektivere Bindung der Partikel an Blutgerinnselkomponenten erreichbar. Die spezifische Bindefähigkeit der Substanzen an Komponenten eines sich bildenden oder in Auflösung befindlichen Blutgerinnsel ist eine wichtige Voraussetzung für das Funktionieren des erfindungsgemäßen Verfahrens. Ob die einzusetzenden Substanzen diese Eigenschaft per se aufweisen oder ob bindungsvermittelnde Komponenten benötigt werden, läßt sich durch Bindungsteste - wie im folgenden am Beispiel von partikelgebundenen Substanzen dargestellt - feststellen:

Die Auswahl geeigneter Partikel als bindungsvermittelnde Komponenten kann durch Prüfung der Bindung der Partikel an eine immobilisierte Fibrinoberfläche erfolgen. Eine solche Fibrinoberfläche kann beispielsweise nach Masson & Angles-Cano (Biochem. J. (1988) 256: 237-244) wie folgt hergestellt werden, in kürze: Gefäße aus Polyvinylchlorid werden mit einer 2,5%-igen (v/v) Glutaraldehyd Lösung in 0,1 M Natrium-Bicarbonat-Puffer (pH 9,5) für 2 Stunden bei Raumtemperatur voraktiviert und dann wird eine Fibrinogen-haltige Lösung appliziert (0,3 µmol/l in 0,1 M NatriumPhosphat-Puffer (pH 7,4) beinhaltend 1 mmol/l Calciumchlorid). Bei Verwendung von handelsüblichen, etwa durch Gamma-Bestrahlung voraktivierten Polystyrol-Röhrchen oder Mikrotitrationsplatten für die Proteinbeschichtung können die verwendeten Testgefäße direkt mit einer Fibrinogen-haltigen Lösung in Kontakt gebracht werden. Nach mehrstündiger Inkubation, üblicherweise über Nacht (ca. 18 Stunden), wird nicht gebundenes Fibrinogen durch Waschen mit einem Detergenz-haltigen Waschpuffer (z.B. aus der Enzygnost-Linie der Dade Behring Marburg GmbH) entfernt. Das gebundene Fibrinogen wird durch Zusatz von Thrombin (1 NIH Einheit/ml in 50 mM Tris-HCl; 1 mM CaCl₂; pH 7,4; beispielsweise Test-Thrombin Reagenz; Dade Behring Marburg GmbH) in Fibrin umgewandelt und anschließend das Thrombin durch Waschen, z.B. mit einer Lösung beinhaltend 0,5 M NaCl; 8 mM CaCl₂ und 0,05% Tween 20, entfernt. Nach einem weiteren Waschschritt mit einem üblichen Detergenz-haltigen Waschpuffer werden freie Bindungsstellen auf der Fibrin-Festphase durch einmaliges Waschen mit einer 0,2%-igen (w/v) Rinderserumalbumin-Lösung abgesättigt. Die Prüfung der Bindung der zu untersuchenden Partikel - z.B. Latexpartikel mit Photosensitizem (Sensitizer-Partikel) in Kombination mit Latexpartikeln mit chemilumineszierenden Verbindungen (Chemiluminescer-Partikel) (s.a. Clin. Chem. (1996) 42:1518-1526) - an die Fibrinoberfläche erfolgt durch Zugabe der Partikel in die beschichteten Testgefäße in einem neutralen Puffer (beispielsweise: 50 mM Tris-HCl; 0,9% NaCl; 0,05% Tween 20; pH 7,4). Nach einer Inkubation von 10 min (je nach Untersuchungsmaterial sind auch kürzere oder längere Inkubationszeiten empfehlenswert) wird das mit Fibrin beschichtete Testgefäß mit dem gleichen Puffer ein- oder mehrmals gewaschen und anschließend der Energietransfer zwischen den im gewaschenen Teströhrchen haftenden Sensitizer- und Chemiluminescer-Partikel in einem geeignetem Meßgerät, z.B. wie im Beispiel 1 näher beschrieben, gemessen. Zum Ausschluß von Partikeln mit der unerwünschten Eigenschaft auch an Fibrinogen zu binden, kann die Inkubation der Partikel in Gegenwart steigender Konzentrationen an Fibrinogen erfolgen. Bei Fibrinogen-bindenden Partikeln wird durch das bis zu einer Konzentration von 5 g/l zugesetzte Fibrinogen die Bindung an die Fibrinbeschichtete Oberfläche deutlich reduziert und damit das Meßsignal letztendlich verringert. In Analogie zu diesem Verfahren können auch andere für das erfindungsgemäße Verfahren geeignete bindungsvermittelnde Komponenten ausfindig gemacht werden.

Eine besonders bevorzugte Ausführungsform des erfindungsgemäßen Verfahrens ist dadurch gekennzeichnet, daß als Substanzen Photosensitizer, beispielsweise Aceton, Benzophenon, 9-Thioxanthone, Eosin, 9,10-Dibromoanthrazen, Chlorophyll, Buckminsterfullerene, Methylen Blau, Rose Bengal, Porphyrine, Phthalocyanine und/oder deren Derivate, und chemilumineszierende Verbindungen, beispielsweise Olefine, 9-Akylidenexanthane, 9-Alkylidene-N-alkylakridane, Enolether, Enamine, Arylvinylether, Dioxene, Arylimidazole und/oder Lucigenin eingesetzt werden und der vom Photosensitizer generierte Singulett-Sauerstoff die chemilumineszierenden Verbindungen zur Lichtaussendung aktivieren kann. Bevorzugt werden im erfindungsgemäßen Verfahren auch die Verwendung von Stoffen wie z. B. Luminol und Oxalatester, die mit Singulett-Sauerstoff zu Intermediaten reagieren, welche mit dem Fachmann bekannten Reagenzien unter Lichtaustrahlung reagieren können.

Die chemilumineszierenden Verbindungen emmittieren in der Regel Licht in der Wellenlängenbereichen über 300 nm. Die Fluoreszenz von Plasma sinkt rapide im Bereich von 500 nm und kann über 550 nm vemachlässigt werden. Werden höhere Wellenlängen gefordert, können die chemilumineszierenden Verbindungen erfindungsgemäß auch mit Fluorophoren in Kontakt gebracht werden, die von den aktivierten chemilumineszierenden Verbindungen angeregt werden können und bei höheren Wellenlängen emittieren. Geeignete Fluophore sind z.B. Rhodamine, Ethidiumbromid, 5-Dimethylaminonapthalen-1-sulfonyl, Europiumchelate mit dem Agenz 3-(2-thienoyl)-1,1,1-trifluoroaceton [Eu(TTA)₃ (TTA = 3-(2-thienoyl)-1,1,1-trifluoroaceton)] oder Rutheniumchelate mit dem Agenz 2,2'-dipyridyl [Ru(bpy)₃⁺⁺ (bpy = 2,2'-dipyridyl)].

Eine weitere bevorzugte Ausführungsform des erfindungsgemäßen Verfahrens ist, dadurch gekennzeichnet, daß als Substanzen Photosensitizer und fluoreszierende Verbindungen eingesetzt werden und der vom Photosensitizer generierte Singulett-Sauerstoff die fluoreszierende Verbindung zur Lichtaussendung aktivieren bzw. in einem Quenchprozess die Lichtaussendung unterdrücken kann. Insbesondere werden erfindungsgemäße Verfahren bevorzugt, die durch den Einsatz fluoreszierender Verbindungen gekennzeichnet sind, die durch Reaktion mit Singulett-Sauerstoff einer Photooxidation - einem "Photobleaching" - unterliegen, wie zum Beispiel 1,3-di-Phenylisobenzofuran, oder mit Singulett-Sauerstoff als photoaktive Vorstufen zu Fluorophoren reagieren, wie zum Beispiel Oxenumbelliferylether oder Umbelliferylselenide.

Hinsichtlich weiterer Beispiele für für das erfindungsgemäße Verfahren geeignete Partikel, Photosensitizer, chemilumineszierende oder fluoreszierende Verbindungen wird insbesondere auf EP 0 515 194, Ullman et al. (Proc. Natl. Acad. Sci. 91:5426-5430, 1994) u. Ullman et al. (Clinical Chemistry 42:1518-1526, 1996, WO 95/06877) verwiesen.

Das erfindungsgemäße Verfahren ist einsetzbar zum Nachweis beispielsweise
- eines genetisch-bedingten und/oder erworbenen Mangels an Faktoren des Blutgerinnungssystems, wie beispielsweise von Willebrand-Faktor, Faktoren (Faktor = F) des extrinsischen Systems wie FVII, FX, FII, FV oder Protein Z, Faktoren des intrinisischen Systems wie FXII, Präkallikrein, hochmolekulares Kininogen, FXI, FIX oder FVIII, oder Faktoren der Kontrollsysteme wie Antithrombin III, tissue factor pathway inhibitor, Protein C oder Protein S, C1-Inhibitor,
- eines genetisch-bedingten und/oder erworbenen Mangels an Faktoren des Fibrinolysesystem wie beispielsweise des Gewebs- oder urinären Plasminogenaktivators, des Plasminogens, des α,2-Antiplasmins, der Plasminogen-Aktivator-Inhibitoren 1, 2 und 3 oder des Thrombin-aktivierbaren Fibrinolyse Inhibitors (TAFI),
- von genetischen Defekten der Thrombozyten wie beispielsweise des Bemard-Soulier-Syndroms, der Thrombasthenie Glanzmann, des Dense-Body-Mangels, des α-Granula-Mangels oder von Thromboxan-Synthese-Defekten,
- erworbenen Hämostasestörungen z.B. infolge von Krankheiten oder Krankheitszuständen wie Urämie, myeloproliferative Erkrankungen, solide Tumore, der storage-pool disease oder Entzündungen, insbesondere solche Entzündungen, die zu proteolytisch oder oxidativ veränderten Proteinen wie zum Beispiel des Protein C Systems, des Antithrombin III und/oder des Plasminogen-Aktivator-Inhibitors 1 führen,
- erworbenen Hämostasestörungen infolge von verabreichten Therapeutika, die die Aktivität einzelner oder mehrerer Komponenten des Hämostasesystems beeinflussen, z.B. Heparin, Coumarin-Derivate, Hirudin, Kontaktphase-Inhibitoren wie z.B. boviner Trypsin Inhibitor oder C1-Esterase Inhibitor, Aspirin oder Antikörper gegen Plättchenrezeptoren,
- oder von genetischen und/oder erworbenen Defekten des Komplementsystems.

Mit dem erfindungsgemäßen Verfahren kann auch die Zeit bis zur Bildung eines Fibringerinnsels bestimmt werden. In einer Ausführungsform des erfindungsgemäßen Verfahrens wird die Gerinnselbildung in einem Plasma oder in einem zumindest Fibrinogen und/oder Blutplättchen enthaltenden Medium ausgelöst durch den Zusatz von die Gerinnung auslösenden Substanzen,
- wie Enzyme z.B. aus Schlangengiften oder Thrombin, oder anderen aktiven Proteasen
- oder durch Oberflächen-aktive Substanzen, wie Silicate oder Phenolderivate,
- oder durch aktivierte Blutplättchen oder Blutplättchen aktivierende Substanzen, wie z.B. Thrombin, Collagen, Adrenalin, Adenosin-Diphosphat,
- oder durch optionalen Zusatz Gerinnungs-unterstützender Substanzen, wie Puffersubstanzen, Calciumchlorid und/oder Phospholipide,
- oder einer oder mehrerer dieser Substanzen.

Das erfindungsgemäße Verfahren läßt sich nutzen, um die aktivierte partielle Thromboplastinzeit (APTT), die Thromboplastinzeit (PT), die Protein C Aktivierungszeit (PCAT), die Russell's Viper Venom Zeit (RVVT) oder die Thrombinzeit zu bestimmen.

Die aktivierte, partielle Thromboplastinzeit (APTT) erfaßt Störungen des intrinsischen Weges des humoralen Gerinnungssystems. Die Thromboplastinzeit erfaßt Störungen des extrinsischen Weges des humoralen Gerinnungssystems (s.a. Colman RW et al. Overview on Hemostasis. In: Colman RW, Hirsh J, Marder VJ, Salzman EW, eds., Hemostasis and Thrombosis, J.B. Lippincott Company, 3rd Edition, 1994; Seiten 3-18). Teste zu deren Bestimmung sind Suchteste, die jeweils alle Komponenten des jeweiligen Astes des humoralen Gerinnungssystems bis zur Bildung des Thrombins, das Fibrinogen zum Fibringerinnsel umsetzt, benötigen.

In einer bevorzugten Ausführungsform des erfindungsgemäßen Verfahrens wird zur Erfassung der Faktoren des Fibrinolysesystem die zu messende Probe mit den zur Gerinnselbildung notwendigen Faktoren substitutiert, wie etwa durch Mischen mit einem Mangelplasma oder dem Zusatz von gereinigten Faktoren wie z.B. urinärer Plasminogenaktivator (uPA), Gewebsplasminogenaktivator (tPA), α₂-Antiplasmin, Plasminogen, Plasminogen-Aktivator Inhibitor 1 und/oder Faktor XII, insbesondere von Fibrinogen. Bei bestimmten Ausführungsformen des erfindungsgemäßen Verfahren kann eine spontane Lyse oder eine Lyse nach Zusatz von Aktivatoren, wie beispielsweise Faktor XIIa, tPA, uPA oder Streptokinase, oder direkt nach Zusatz von Plasmin ausgelöst werden.

In einer besonders bevorzugten erfindungsgemäßen Ausführungsform wird der Zeitpunkt des Beginns der Lyse eines Fibringerinnsels durch einen Signalanstieg ermittelt: Ein Plasma wird zur Gerinnung gebracht. Durch Zugabe von z.B. Streptokinase, einem Plasmin aktivierenden Enzym aus *Streptococcus,* wird das Gerinnsel lysiert. Dies zeigt sich in herkömmlichen Verfahren in einer kontinuierlichen Abnahme des Meßsignals. Der Beginn der Auflösung des Gerinnsels ist jedoch schwer festzustellen, da an optisch messenden Geräten die Signalabnahme schleichend erfolgt. Überraschenderweise wird mit dem Beginn der Lyse beim erfindungsgemäßen Verfahren das Signal plötzlich und sehr stark erhöht. Dies ist nicht auf Störungen der Testkomponenten durch die zugesetzte Streptokinase zurückführbar, da diese von Beginn der Meßzeit an anwesend ist.

Dieser gefundene überraschende Effekt bietet die neuartige Möglichkeit den Beginn einer Lyse sehr genau zu bestimmen. Dies erweitert die Möglichkeit der Diagnostik des fibrinolytischen Systems, etwa zur Bestimmung von einzelner Faktoren als auch des gesamten oder der durch Faktor XIIa, uPA oder tPA vermittelten Fibrinolyse (Bachmann F. The plasminogen-Plasmin Enzyme System. In: Colman RW, Hirsh J, Marder VJ, Salzman EW, eds., Hemostasis and Thrombosis, J.B. Lippincott Company, 3rd Edition, 1994; Seiten 1592-1622). Dies war bisher vor allem daran gescheitert, daß eine exakte Messung der Lysezeit vergleichbar zur Gerinnungszeit nicht möglich war.

Umfaßt vom erfindungsgemäßen Verfahren werden auch Ausführungsformen zur Bestimmung der Aktivität von Blutplättchen über deren Aggregationsfähigkeit, insbesondere auch Ausführungsformen bei denen die Blutplättchenaggregation durch Stoffe wie Adenosindiphosphat, Kollagen, Thrombin, Serotonin oder Epinephrin, lonophore, Komplement oder Streptolysin ausgelöst werden kann.

Weitere Ausführungsformen des erfindungsgemäßen Verfahrens können zur Ermittlung der Konzentration - insbesondere im Blut oder Plasma - oder der Wirksamkeit von Arzneimitteln wie beispielsweise
- Heparin, tPA, Acetylsalicylsäure, Coumarin-Derivate, Hirudin, Streptase
- Plasmafaktorenkonzentrate wie z.B. Prothrombin-Komplexe, Faktor VIII-Konzentrat oder vWF-haltige Faktorenkonzentrate,
- Antikörper gegen Plättchenrezeptoren wie beispielsweise GP IIb/IIIa-Komplex, GP Ib/V/X-Komplex, Serotonin-Rezeptor, Adenosindiphosphat-Rezeptor oder Thrombin-Rezeptor,
- Kontaktphaseinhibitoren wie z.B. boviner Trypsin Inhibitor oder C1-EsteraseInhibitor,
- Inhibitoren des Gerinnungssystem wie z.B. inaktivierter Faktor VIIa, Antikörper gegen Gewebsthromboplastin, tissue factor pathway inhibitor, Antithrombin III, Thrombomodulin oder aktiviertes Protein C,
- sowie Fibrinaggregationshemmem verwendet werden.

Sie sind auch einsetzbar für die therapeutische Arzneispiegelüberwachung von beispielsweise Heparin, tPA, Acetylsalicylsäure, Coumarin-Derivaten, Hirudin, Kontaktphase-Inhibitoren wie z.B. boviner Trypsin Inhibitor oder C1-Esterase . Inhibitor, oder Antikörpern gegen Plättchenrezeptoren oder an Plättchen assozierten Proteinen wie z.B. Heparin/Plättchenfaktor 4-Komplex.

Die Erfindung umfaßt auch die Verwendung von Diagnosemitteln, enthaltend einen oder mehrere Substanzen, die infolge einer Blutplättchenaggregation, einer Gerinnselbildung und/oder einer Gerinnselauflösung in einen Abstand zueinander gebracht werden, der eine Wechselwirkung, insbesondere einen Energietransfer, zwischen den Substanzen erlaubt oder unterbindet, wobei mindestens eine der Substanzen direkt oder indirekt unspezifisch, z.B. durch hydrophobe oder elektrostatische Wechselwirkung, an Komponenten eines sich bildenden oder auflösenden Blutgerinnsels binden kann und eine oder mehrere der Substanzen an suspendierbare Partikel kovalent, über spezifische Wechselwirkung und/oder adsorbtiv gebunden sind und/oder in diese eingelagert sind oder selbst suspendierbare Partikel darstellen oder ein Teil derselben sind, zur Durchführung des erfindungemässen Verfahrens. Dieses Diagnosemittel kann zum Nachweis einer erworbenen oder vererbten Hämostasestörung eingesetzt werden.

Im folgenden wird die Erfindung anhand von Beispielen und mit Bezug auf die beiliegenden Abbildung näher erläutert.

In den Abbildungen werden u.a. folgende Abkürzungen verwendet: "t" = Meßzeit in Sekunden ("s"); Meßsignale: "A 405 nm" = Absorptionseinheiten in "mE" bei 405 nm Wellenlänge; "c" = Counts als Meßsignal.

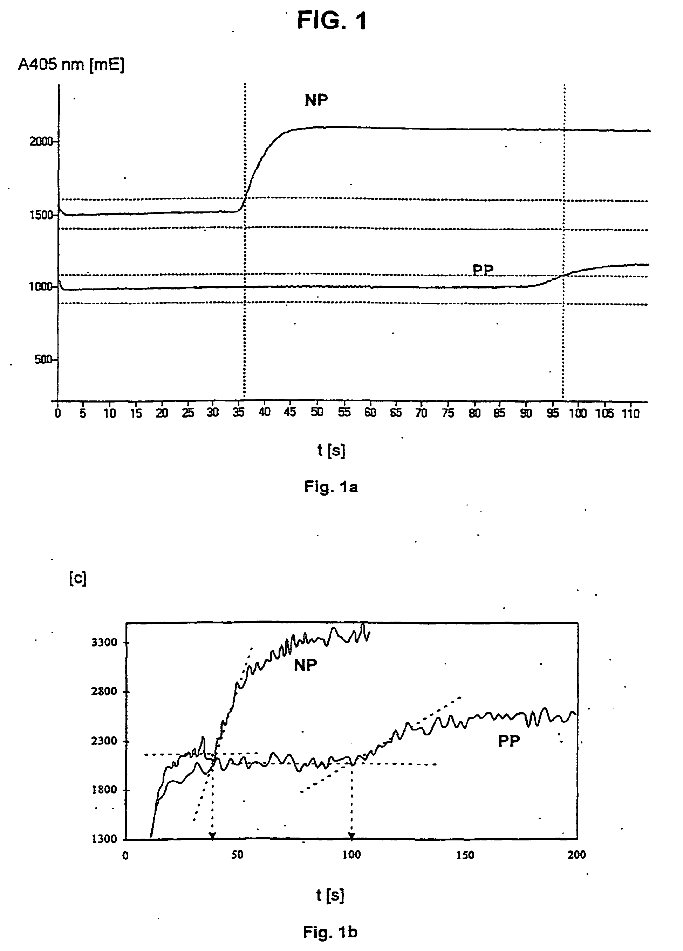

**FIG. 1** zeigt den Reaktionsverlauf einer APTT eines normalen Plasmas (NP) und eines pathologischen Plasmas (PP) im herkömmlichen turbidimetrischen Verfahren **(Fig. 1a)** sowie im erfindungsgemäßen Verfahren **(Fig. 1b).** Die Ermittlung der Gerinnungszeit durch die Bestimmung des Schnittpunktes aus der jeweiligen Basislinie und der Gerinnselbildungslinie ist schematisch dargestellt

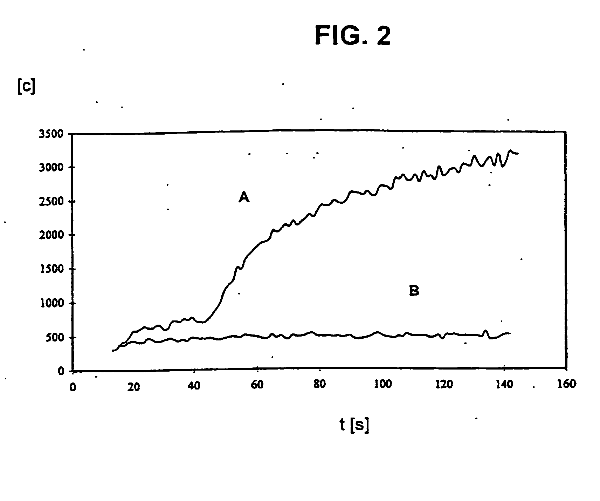

**FIG. 2** zeigt die Bestimmung der APTT einer Vollblutprobe im erfindungsgemäßen Verfahren (A). Zur Kontrolle wurde der Test ohne Zugabe von Calciumchlorid durchgeführt (B).

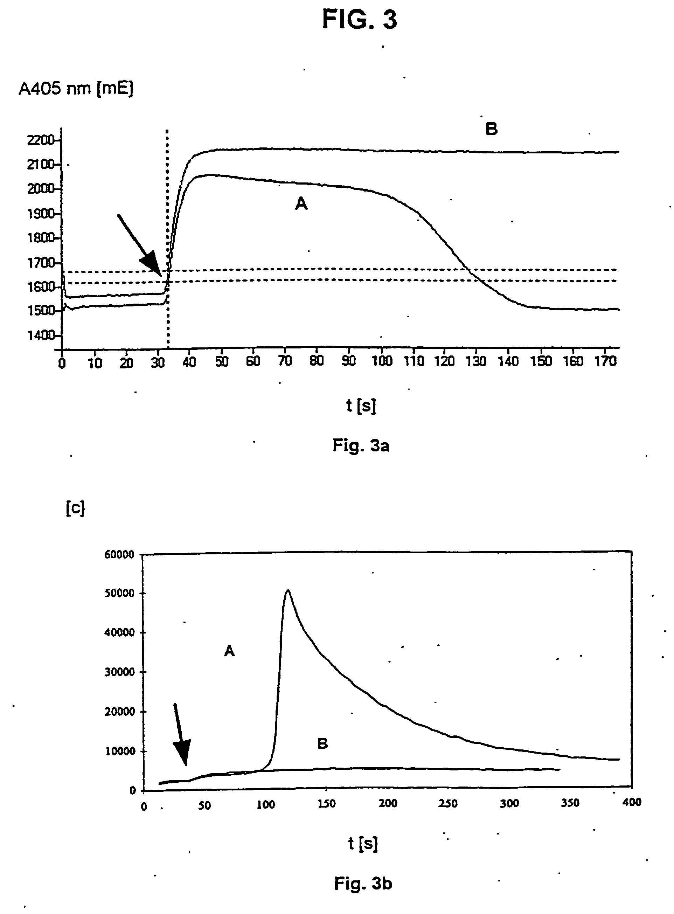

**FIG. 3** zeigt den Reaktionsverlauf einer APTT eines normalen Plasmas im herkömmlichen turbidimetrischen Verfahren **(Fig. 3a)** sowie im erfindungsgemäßen Verfahren **(Fig. 3b)** unter Zusatz von Streptokinase zur Lyse des Fibringerinnsels (A). Zum Vergleich ist der Reaktionsverlauf ohne Streptokinase mit angegeben (B). Der Zeitpunkt der Gerinnselbildung ist jeweils mit einem Pfeil gekennzeichnet.

Die folgenden Beispiele dienen der exemplarischen Erläuterung des erfindungsgemäßen Verfahrens: Am Beispiel von Partikel-gebundenen Substanzen nach dem Verfahren von Ullman et al. (Clinical Chemistry (1996) 42:1518-1526) wurden die wichtigsten grundlegenden Teste der Hämostase-Diagnostik exemplarisch durchgeführt. Diese Verfahren werden in verschiedenen Abwandlungen als Such-, aber auch als Einzelfaktoren-Test angewandt, beruhen letztlich aber immer auf dem gleichen Grundprinzip, nämlich der Bestimmung der Zeit bis ein meßbares Gerinnsel entstanden ist bzw. sich dieses wieder aufgelöst hat.

Soweit nicht anders angegeben wurden für die Hämostase-Teste Reagenzien der Dade Behring Marburg GmbH, Marburg, Deutschland, verwendet.

### Beispiel 1

### Herstellung von Sensitizer- bzw. Chemiluminescer-Partikel

Die Herstellung von Sensitizer- und Chemiluminescer-Partikeln ist im Detail in EP 0 515 194, Clin. Chem. (1996) 42:1518-1526 und Proc. Natl. Acad. Sci. (1994) 91:5426-5430 beschriebenen. Die Partikel können z.B. Dextranumhüllungen tragen und zusätzlich gebundes Streptavidin aufweisen (s.a. Proc. Natl. Acad. Sci. (1994) 91:5426-5430). Exemplarisch ist im folgenden die Herstellung einer Variante von Sensitizer- und Chemiluminescer-Partikeln beschrieben (s.a. EP 0 515 194, Beispiel 8, für weitere Details):

### Herstellung von Sensitizer-Partikeln:

Eine Lösung von Chlorophyll-a in Benzylalkohol (1,0 ml; 0,6 mM) wird zu 8,0 ml Benzylalkohol gegeben, der auf 105°C erhitzt worden ist. Eine Suspension von Latex-Beads (175 nm, Carboxyl-modifiziertes Latex, Bangs Laboratories, Carmel, IN) in Wasser (10%; 1,0 ml) wird zu der Benzylalkohol-Lösung gegeben. Das Gemisch wird für 5 Minuten bei 105°C gerührt und dann auf Raumtemperatur gekühlt. 10 ml Ethanol werden hinzugegeben und das Gemisch zentrifugiert. Das Pellet wird in einem 1:1 Wasser-Ethanol Gemisch (10 ml) resuspendiert und anschließend wieder zentrifugiert. Das gleiche Verfahren wird mit Wasser wiederholt und anschließend das Pellet in physiologischer Kochsalz-Lösung aufgenommen.

### Herstellung der Chemiluminescer-Partikel (= Acceptor-Partikel):

20 ml der carboxyl-modifizierten Latex-Partikel-Suspension (10% Suspension in Wasser) werden mit 20 ml 2-Ethoxyethanol gemischt Die Mischung wird auf 90°C erhitzt. 20 ml einer Lösung aus 10 mM Dioxen, 20 mM Europiumchelat mit dem Agenz 3-(2-thienoyl)-1,1,1trifluoroaceton (Kodak, CAS # 14054-87-6) (EuTTA) und 60 mM Trioctylphosphinoxid (TOPO) in 2-Ethoxyethanol werden der Partikel-Suspension zugegeben. Das Gemisch wird weiter bei 97°C für 7 Minuten erhitzt. Nach dem Abkühlen auf Raumtemperatur werden 40 ml Ethanol zugegeben und das Gemisch zentrifugiert. Das Pellet wird dann in 80% Ethanol resuspendiert und zentrifugiert. Dieser Waschprozess wird mit 10% Ethanol wiederholt. Zum Abschluß werden die Partikel in physiologischer Kochsalzlösung aufgenommen.

### Beispiel 2

### Bestimmung der aktivierten, partiellen Thromboplastin-Zeit (APTT) im herkömmlichen (Turbidimetrie) und im erfindungsgemäßen Verfahren.

Herkömmliches Verfahren: Ein Normalplasma Pool (Control Plasma N; Prod. Nr. ORKE), sowie ein pathologisches Plasma (Pathoplasma II; Prod. Nr. OTXK) wurde parallel an einem herkömmlichen, optisch messenden Gerinnungsautomaten (Behring Coagulation Timer, BCT) mit Pathromtin SL (Prod. Nr.OQGS) gemessen. Hierzu wurden 50 µl Probe mit 50 µl Pathromtin SL gemischt und nach 2-minütiger Inkubation bei +37°C die Gerinnung durch Zugabe von 50 µl 25 mM Calciumchloridlösung (Prod. Nr. ORHO) gestartet.

Die Bestimmung der aktivierten, partiellen Thromboplastin-Zeit im erfindungsgemäßen Verfahren erfolgte mit Patromthin Gebrauchslösung, hergestellt aus Kaolin-Aktivator (Prod. Nr. OTXC) und Phospholipid (Prod. Nr. OTXB) nach Angaben des Herstellers, sowie 25 mmol/l Calciumchlorid-Lösung (Prod. Nr. ORHO). Die Bestimmung erfolgte an einem Luminometer. Die Meßeinheit ist ausgestattet mit einem 670 nm Diodenlaser (30 mW Lichtquelle) und einer Photonenzähleinheit (Hamamatsu R 4632 Photomultipliertube). Vor den Photomultiplier wurde eine Filterkombination aus einem Kurzpassfilter und einem Langpassfilter gesetzt, die eine Lichtmessung zwischen 550 nm und 650 nm erlaubt. Das gesamte System wurde vor Umgebungslicht geschützt. Ein typischer Meßvorgang beinhaltet eine Illuminationszeit mit Hilfe der Lichtquelle und eine anschließende zweite Phase, in der die aus dem Meßgut ausgesendeten Photonen gezählt werden. Dieser Meßvorgang kann beliebig oft wiederholt werden, abhängig von der gewünschten Zeit, in welcher das Meßgut beobachtet werden soll. Die illuminationszeit beträgt typischerweise 0,1 - 3 sec. Die Meßzeit kann in demselben Bereich liegen. Zwischen zwei Meßvorgängen kann eine Phase ohne Illumination bzw. Messung liegen, die abhängig von der Anzahl der notwendigen Meßpunkte gewählt werden kann. Zusätzlich wurden erfindungsgemäß an Partikel gebundene Substanzen verwendet:
- Chemiluminescer-Partikel = Acceptor-Partikel: 2,5 mg/ml in physiologischer Kochsalzlösung;
- Sensitizer-Partikel: 2,5 mg/ml in physiologischer Kochsalzlösung.

Folgende Proben wurden gemessen:
1 Normalplasma Pool (Control Plasma P, Prod. Nr. OUPZ), 2 pathologische Plasmen, die durch Absorption der Vitamin K-abhängigen Gerinnungsenzyme hergestellt werden (Pathoplasma I, Prod. Nr. ORXK; Pathoplasma II, Prod. Nr. OTXL), 1 pathologisches Plasma, das durch Verdünnung aller Gerinnungsfaktoren hergestellt wurde (Control Plasma P; Prod. Nr. OUPZ), sowie ein Serum eines gesunden Blutspenders.

Die Reagenzien wurden vor Gebrauch auf +37 °C vorgewärmt. Zur Testdurchführung wurden die Komponenten wie folgt gemischt:
200 µl Probe
100 µl Acceptor-Partikei
25 µl Sensitizer-Partikel
200 µl Pathromtin Gebrauchslösung
Anschließend wurde 2 min bei +37 °C inkubiert und die Gerinnung durch Zugabe von
200 µl 25 mM Calciumchlorid-Lösung
gestartet. Die Signale wurden über den Verlauf der Zeit aufgezeichnet.

Aus denen im erfindungsgemäßen Verfahren erhaltenen Kurvenverläufen wurde die Gerinnungszeiten ermittelt, indem jeweils für die Basislinie und die ersten 10 Sekunden der Reaktionskurve eine lineare Regression durchgeführt wurde. Der Schnittpunkt beider Linien wurde als Gerinnungszeit gewertet (s.a. Fig. 1 b).

Ergebnis: Tabelle 1 zeigt, daß die im erfindungsgemäßen Verfahren bestimmten Gerinnungszeiten der Plasmen im vom Hersteller mit klassischen (mechanisch und optisch) Verfahren ermittelten Sollwertbereich liegen. Mit Serum wurde kein Signal erhalten. In Serum sind zwar die Gerinnungsenzyme des jeweiligen Astes der Gerinnung aktiv, das Fibringerinnsel ist jedoch entfernt worden. Dies zeigt, daß im erfindungsgemäßen Verfahren die Entstehung des Gerinnsels, nicht jedoch eine irgendwie geartete Wirkung eines Gerinnungsenzyms auf die zugegebenen Reagenzien für die Entstehung des Signals ursächlich ist.

**Tabelle 1 Im erfindungsgemäßen Verfahren (CECA) erhaltenen Gerinnungszeiten der APTT im Vergleich zu den vom Hersteller mittels klassischer (mechanischer und optischer) Verfahren ermittelten Sollwerte verschiedener kommerzieller Plasmen. Angaben in sec.**

| **Probe** | **CECA** | **angegebener Sollwertbereich** |
|---|---|---|
| Control Plasma N | 39,2 | 30,1 - 40,7 |
| Control Plasma P | 81,9 | 57,6 - 86,4 |
| Pathoplasma I | 67,1 | 51,2 - 76,8 |
| Pathoplasma II | 99,0 | 67-101 |
| Serum | keine | |
| | Gerinnung | |

### Beispiel 3

### Bestimmung der Thromboplastin-Zeit (PT) im herkömmlichen (Turbidimetrie) und im erfindungsgemäßen Verfahren.

Die Bestimmung der PT erfolgte unter Verwendung von Thromborel S (Prod. Nr. OUHP) nach Angaben des Herstellers mit den unter Beispiel 2 genannten Proben am optisch messenden Gerinnungsgerät im klassisch-optischen Verfahren und am Luminometer im erfindungsgemäßen Verfahren.

Am BCT (turbidimetrische Messung) wurde je 50 µl Pathoplasma I bzw. Pathoplasma II in ein Meßkammer pipettiert und durch Zugabe von 100 µl Thromborel S die Gerinnung gestartet.

Am Luminometer wurden zur Testdurchführung die Komponenten wie folgt gemischt:
200 µl Probe
100 µl Acceptor-Partikel
25 µl Sensitizer-Partikel.
Mit Zugabe von
400 µl Thromborel S
wurde die Gerinnung gestartet. Die Signale wurden über den Verlauf der Zeit aufgezeichnet. Es zeigten sich praktisch identische Verläufe der Reaktionkinetik, wobei im herkömmlichen Verfahren eine Trübungszunahme gemessen werden, im erfindungsgemäßen Verfahren eine Zunahme der Chemilumineszenz-Emission.

Wie in Beispiel 2 beschrieben wurden die Reaktionsverläufe, die im erfindungsgemäßen Verfahren erhalten wurden, analysiert. Die Ergebnisse im Vergleich zu den in herkömmlichen Verfahren zu erwartenden Gerinnungszeiten gemäß Herstellerangaben sind in Tabelle 2 aufgeführt. Es zeigt sich ebenso wie in Beispiel 2 eine gute Übereinstimmung. Mit Serum als Probe wurde aus dem in Beispiel 2 genannten Grund keine Änderung des Meßsignals erhalten.

**Tabelle 2 Im erfindungsgemäßen Verfahren (CECA) erhaltenen Gerinnungszeiten der PT im Vergleich zu den vom Hersteller mittels klassischer (mechanischer und optischer) Verfahren ermittelten Sollwerte verschiedener kommerzieller Plasmen. Angaben in sec.**

| **Probe** | **CECA** | **angegebener Sollwertbereich** |
|---|---|---|
| Control Plasma N | 14,7 | 9,5 - 14,3 |
| Control Plasma P | 30,7 | 18,8 - 28,2 |
| Pathoplasma I | 37,0 | 24,6 - 36,8 |
| Pathoplasma II | 56,0 | 40,7 - 61,1 |
| Serum | keine | |
| | Gerinnung | |

### Beispiel 4

### Bestimmung der Gerinnung in Vollblut

Die Bestimmung einer Gerinnungszeit in Vollblut ist in herkömmlichen, optisch messenden Geräte nicht möglich, da die optische Dichte der Probe zu hoch ist. Im erfindungsgemäßen Verfahren wurde die Reaktionskurve einer Vollblutprobe in einer APTT wie folgt aufgezeichnet:

Die Reagenzien wurden vor Gebrauch auf +37 °C vorgewärmt. Zur Testdurchführung wurden die Komponenten wie folgt gemischt:
100 µl Probe (Citratblut)
50 µl Acceptor-Partikel
12,5 µl Sensitizer-Partikel
100 µl Pathromtin Gebrauchslösung
Anschließend wurde 2 min bei +37 °C inkubiert und die Gerinnung durch Zugabe von
100 µl 25 mM Calciumchlorid-Lösung oder physiologischer Kochsalzlösung gestartet. Die Signale wurden über den Verlauf der Zeit aufgezeichnet.

Das Ergebnis ist in FIG. 2 aufgezeigt. Im Vergleich wurde die Reaktionskurve eingetragen (B), die erhalten wird, wenn kein Calciumchlorid zugesetzt wird und somit keine Gerinnungsaktion ausgelöst wird. Das Beispiel zeigt, daß auch mit Vollblut als Probe eine für eine Gerinnung typische Reaktionskurve erhalten wird, die durch die Bildung eines Gerinnsels entsteht und nicht etwa durch unspezifische Seitenreaktionen ausgelöst wird.

Weiterhin zeigt sich durch die Applizierbarkeit des erfindungsgemäßen Verfahrens auf Vollblutproben, daß dieses gegenüber herkömmlichen Verfahren robuster hinsichtlich trübungsbedingten Probestörungen ist.

Die geringere Störanfälligkeit des erfindungsgemäßen Verfahrens gegenüber unspezifischen Trübungen der Probe oder von Reagenzien geht bereits aus Beispiel 2 hervor. Dort wurde am Luminometer eine APTT durchgeführt, bei der als Aktivator der Kontaktphase des intrinsischen Gerinnungssystems Kaolin verwendet wurde (Pathromtin). Kaolin ist ein Tonschichtmineral in Form sübriger Plättchen. Diese erzeugen eine so große Lichtstreuung, daß dieses Reagenz an optisch messenden Geräten nicht eingesetzt werden kann. Deshalb wurde am optisch messenden Gerät mit Pathromtin SL ein anderer, speziell für optisch messende Geräte entwickelter Kontaktphasenaktivator eingesetzt. Es handelt sich um fein gemahlene Siliziumdioxid-Partikel deren unspezifische Lichtstreuung noch tolerierbar ist.

### Beispiel 5

### Bestimmung der Fibrinolyseaktivität im erfindungsgemäßen Verfahren.

Zur Bestimmung der Fibrinolyseaktivität wurde ein normales Plasma (Control Plasma N, Prod. Nr. ORKE) sowie eine Vollblutprobe in der APTT wie unter Beispiel 2 und 4 beschrieben im erfindungsgemäßen Verfahren zur Gerinnung gebracht. Gleichzeitig mit dem Auslösen der Gerinnung wurde durch Zugabe einer Streptokinase haltigen Lösung (100 IU/ml physiologischer Kochsalzlösung; Fa. Hoechst Marion Roussel) auch die Lyse des entstehenden Gerinnsels ausgelöst. Streptokinase ist ein Enzym aus Streptococcus, das mit Plasminogen einen aktiven Komplex eingeht. Dieser Komplex aktiviert des Enzym Plasminogen der Probe zu Plasmin, das Fibrinogen und eben Fibringerinnsel proteolytisch auflöst. In herkömmlichen, optischen Verfahren führt dies zu einem kontinuierlichen Abklingen des Meßsignals (s.a. Fig. 3a).

Die Reagenzien wurden vor Gebrauch auf +37 °C vorgewärmt. Zur Testdurchführung wurden die Komponenten wie folgt gemischt:
100 µl Probe (Control Plasma N, ORKE)
50 µl Acceptor-Partikel
12,5 µl Sensitizer-Partikel
100 µl Pathromtin Gebrauchslösung
Anschließend wurde 2 min bei +37 °C inkubiert und die Gerinnung durch Zugabe von
7,2 µl Streptokinase Lösung (100 IU/ml physiologische Kochsalzlösung)
100 µl 25 mM Calciumchlorid-Lösung
gestartet. Die Signale wurden über den Verlauf der Zeit aufgezeichnet (s. Fig. 3b).

Infolge der einsetzenden Gerinnselbildung kommt es zunächst wie in den Beispielen 2 bis 4 zu einem Anstieg des Meßsignals. Die entsprechende Vergleichskurve ohne Streptokinase ist in Fig. 3b als "B" gekennzeichnet miteingezeichnet In Anwesenheit von Streptokinase kommt es mit Einsetzen der Lyse des Gerinnsels jedoch zu einer plötzlichen Erhöhung des Meßsignals, das in Plasma stärker ausgeprägt ist als in einer ebenfalls gemessenen Vollblutprobe. Dieses Signal klingt dann im zeitlichen Verlauf langsam ab. Dies ist ein bisher nicht beschriebener Effekt. Da Streptokinase von Anfang an präsent ist und keine Änderung des Reaktionsverlaufs bis zum Einsetzen der Gerinnselbildung zu verzeichnen ist, kann dieser plötzliche Signalzuwachs nicht durch Seitenreaktionen der Streptokinase-Lösung verursacht worden sein.

Dieses Beispiel zeigt eine gänzlich neue diagnostische Anwendung des erfindungsgemäßen Verfahrens. Der durch die Gerinnselauflösung bedingte sehr starke und schnelle Signalanstieg - auch im Vollblut - ermöglicht sehr sensitiv und neuartig den Zeitpunkt der Lyse zu bestimmen. In bisherigen Verfahren (s.a. Fig. 3a) wurde nur der ebenfalls hier auftretende Signalabfall beobachtet, der aber in herkömmlichen Verfahren so langsam erfolgt, daß der Eintritt der Lyse bisher nicht exakt meßbar war.

## Patentansprüche

1. Verfahren zum Nachweis einer Hämostasestörung, bei dem infolge einer. Blutplättchenaggregatiön, einer Gerinnselbildung und/oder einer Gerinnselauflösung Substanzen in einen Abstand zueinander gebracht werden, der eine Wechselwirkung, insbesondere einen Energietransfer, zwischen den Substanzen erlaubt oder unterbindet und das Ausmaß an Wechselwirkung gemessen wird, **dadurch gekennzeichnet, daß** eine oder mehrere dieser Substanzen an suspendierbare Partikel kovalent, über spezifische Wechselwirkung und/oder adsorbtiv gebunden sind und/oder in diese eingelagert sind oder selbst suspendierbare Partikel darstellen oder ein Teil derselben sind.

2. Verfahren nach Anspruch 1, **dadurch gekennzeichnet, daß** der Energietransfer mittels kurzlebiger Moleküle, z.B. Singulett-Sauerstoff, Strahlung geringer Reichweite, z.B. radioaktive β-Strahfung, und/oder Energietransfers nach Förster erfolgt.

3. Verfahren nach Anspruch 1 oder 2, **dadurch gekennzeichnet, daß** die Aktivität von Substanzen durch andere Substanzen verstärkt oder inhibiert wird und dies zu einer meßbaren Signaländerung führt wie beispielsweise die Änderung der Intensität oder der Polarisation des ausgestrahlten Lichtes, die Inhibition oder Steigerung von Enzymaktivitäten oder/und die Änderung des Fluoreszenzverhaltens.

4. Verfahren nach einem oder mehreren der Ansprüche 1-3, **dadurch gekennzeichnet, daß** mindestens eine der Substanzen direkt oder indirekt unspezifisch, z.B., durch hydrophobe oder elektrostatische Wechselwirkung, an Komponenten eines sich bildenden oder auflösenden Blutgerinnsels binden kann.

5. Verfahren nach einem oder mehreren der Ansprüche 1-4, **dadurch gekennzeichnet, daß** es sich bei den suspendierbaren Partikeln um z.B. Farbstoffkristalle, Metallsolen, Silica-Partikel, Magnetpartikel, Öltropfen, Lipidpartikel, Dextran, Proteinaggregate oder bevorzugt um Latexpartikel handelt.

6. Verfahren nach einem oder mehreren der Ansprüche 1-5, **dadurch gekennzeichnet, daß** die Oberfläche der Partikel nach deren Herstellung weiter modifiziert wird und/oder die Partikel von einer oder mehreren kovalent oder adsorbtiv gebundenen Schichten oder Schalen - z.B. aus Proteinen, Kohlehydraten, lipophilen Substanzen, Biopolymeren, organischen Polymeren oder Mischungen hiervon - umhüllt sind.

7. Verfahren nach einem oder mehreren der Ansprüche 1-6, **dadurch gekennzeichnet, daß** als Substanzen Photosensitizer und chemilumineszierende Verbindungen eingesetzt werden und der vom Photosensitizer generierte Singulett-Sauerstoff die chemilumineszierenden Verbindungen zur Lichtaussendung aktivieren kann.

8. Verfahren nach einem oder mehreren der Ansprüche 1-6, **dadurch gekennzeichnet, daß** als Substanzen Photosensitizer und fluoreszierende Verbindungen eingesetzt werden und der vom Photosensitizer generierte Singulett-Sauerstoff die fluoreszierende Verbindung zur Lichtaussendung aktivieren bzw. in einem Quenchprozess die Lichtaussendung unterdrücken kann.

9. Verfahren nach einem oder mehreren der Ansprüche 1-8, **dadurch gekennzeichnet, daß** als Photosensitizer Aceton, Benzophenon, 9-Thioxanthone, Eosin, 9,10-Dibromoanthrazen, Chlorophyll, Buckminsterfullerene, Methylen Blau, Rose Bengal, Porphyrine, Phthalocyanine und/oder deren Derivate eingesetzt werden.

10. Verfahren nach einem oder mehreren der Ansprüche 1-9, **dadurch gekennzeichnet, daß** als chemilumineszierende Verbindungen Olefine, 9-Akylidenexanthane, 9-Alkylidene-N-alkylakridane, Enolether, Enamine, Arylvinylether, Dioxene, Arylimidazole und/oder Lucigenin eingesetzt werden.

11. Verfahren nach einem oder mehreren der Ansprüche 1-10, **dadurch gekennzeichnet, daß** chemilumineszierende Verbindungen mit Fluorophoren in Kontakt gebracht werden, die von den aktivierten chemilumineszierenden Verbindungen angeregt werden können und Licht einer höheren Wellenlängen emittieren.

12. Verfahren nach einem oder mehreren der Ansprüche 1-11, **dadurch gekennzeichnet, daß** fluoreszierende Verbindungen eingesetzt werden, die durch Reaktion mit Singulett-Sauerstoff einer Photooxidation (Photobleaching) unterliegen, wie zum Beispiel 1,3-di-phenylisobenzofurän, oder mit Singulett-Sauerstoff als photoaktive Vorstufen zu Fluorophoren reagieren, wie zum Beispiel Oxenumbelliferylether oder Umbelliferylselenide.

13. Verfahren nach einem oder mehreren der Ansprüche 1-12 einsetzbar zum Nachweis eines genetisch-bedingten und/oder erworbenen Mangels an Faktoren des Blutgerinnungs- und Fibrinolysesystem, von genetischen Defekten der Thrombozyten, von krankheitsbedingten oder infolge von verabreichten Therapeutika bedingten Hämostasestörungen und/oder von genetischen und/oder erworbenen Defekten des Komplementsystems.

14. Verfahren nach einem oder mehreren der Ansprüche 1-13, **dadurch gekennzeichnet, daß** die Zeit bis zur Bildung eines .Fibringerinnsels bestimmt wird.

15. Verfahren nach einem oder mehreren der Ansprüche 1-14, **dadurch gekennzeichnet, daß** die Gerinnselbildung in einem Plasma oder zumindest in einem Fibrinogen und/oder Blutplättchen enthaltenden Medium durch den Zusatz von einer oder mehrerer die Gerinnung auslösender Substanzen ausgelöst wird.

16. Verfahren nach einem oder mehreren der Ansprüche 1-15, **dadurch gekennzeichnet, daß** die aktivierte partielle Thromboplastinzeit (APTT), die Thromboplastinzeit (PT), die Protein C Aktivierungszeit (PCAT), die Russell's Viper Venöm Zeit (RVVT) oder die Thrombinzeit bestimmt wird.

17. Verfahren nach einem oder mehreren der Ansprüche 1-16, **dadurch gekennzeichnet, daß** zur Erfassung der Faktoren des Fibrinolysesystem die zu messende Probe mit den zur Gerinnselbildung notwendigen Faktoren substitutiert werden kann, wie etwa durch Mischen mit einem Mangelplasma oder dem Zusatz von gereinigten Faktoren, wie z.B. uPA, tPA, α₂-Antiplasmin, Plasminogen, Plasminogen-Aktivator Inhibitor 1 und/oder Faktor XII, insbesondere von Fibrinogen.

18. Verfahren nach einem oder mehreren der Ansprüche 1-17, **dadurch gekennzeichnet, daß** eine spontane Lyse oder eine Lyse nach Zusatz von Aktivatoren wie beispielsweise Faktor XIIa, tPA, uPA, Streptokinase oder direkt nach Zusatz von Plasmin ausgelöst wird.

19. Verfahren nach einem oder mehreren der Ansprüche 1-18, **dadurch gekennzeichnet, daß** der Zeitpunkt des Beginns der Lyse eines Fibringerinnsels durch einen Signalanstieg ermittelt wird.

20. Verfahren nach einem oder mehreren der Ansprüche 1-19, **dadurch gekennzeichnet, daß** die Aktivität der Blutplättchen über deren Äggregationsfähigkeit bestimmt wird.

21. Verfahren nach einem oder mehreren der Ansprüche 1-20, **dadurch gekennzeichnet, daß** die Blutplättchenaggregation durch Stoffe wie Adenosindiphosphat, Kollagen, Thrombin, Serotonin oder Epinephrin, lonophore, Komplement oder Streptolysin ausgelöst werden kann.

22. Verfahren nach einem oder mehreren der Ansprüche 1-21, **dadurch gekennzeichnet, daß** die Konzentration oder die Wirksamkeit von Arzneimitteln ermittelt wird.

23. Verfahren nach Anspruch 22, **dadurch gekennzeichnet, daß** es zur therapeutischen Arzneispiegelüberwachung eingesetzt wird.

24. Verwendung eines Diagnosemittels, enthaltend eine oder mehrere Substanzen, die infolge einer Blutplättchenaggregation, einer Gerinnselbildung und/oder einer Gerinnselauflösung in einen Abstand zueinander gebracht werden, der eine meßbare Wechselwirkung, insbesondere einen Energietransfer, zwischen den Substanzen erlaubt oder unterbindet, wobei mindestens eine der Substanzen direkt oder indirekt unspezifisch, z.B. durch hydrophobe oder elektrostatische Wechselwirkung, an Komponenten eines sich bildenden oder auflösenden Blutgerinnsels binden kann und eine oder mehrere der Substanzen an suspendierbare Partikel kovalent, über spezifische Wechselwirkung und/oder adsorbtiv gebunden sind und/oder in diese eingelagert sind oder selbst suspendierbare Partikel darstellen oder ein Teil derselben sind , zur Durchführung des Verfarhens nach einem oder mehreren der Ansprüche 1-23 .

25. Verwendung eines Diagnosemittels gemäß Anspruch 24 zum Nachweis einer erworbenen oder vererbten Hämostasestörung.

## Claims

1. A method for detecting a disturbance of hemostasis, in which, as a consequence of blood platelet aggregation, clot formation and/or clot dissolution, substances are brought to a distance from each other which permits or prevents an interaction, in particular an energy transfer, between the substances, and the extent of the interaction is measured, wherein one or more of these substances are bound covalently, by way of a specific interaction, and/or adsorptively to suspendable particles, and/or are incorporated into these particles or themselves constitute suspendable particles or a part thereof.

2. The method as claimed in claim 1, wherein the energy transfer takes place by means of short-lived molecules, e.g. singlet oxygen, short-range radiation, e.g. radioactive β radiation, and/or energy transfer in accordance with Förster.

3. The method as claimed in claim 1 or 2, wherein the activity of substances is augmented or inhibited by other substances and this leads to a measurable change in signal, for example change in the intensity or polarization of the emitted light, inhibition of or increase in enzyme activities and/or change in fluorescence behavior.

4. The method as claimed in one or more of claims 1-3, wherein at least one of the substances is able to bind nonspecifically, e.g. by means of hydrophobic or electrostatic interaction, directly or indirectly, to components of a blood clot which is forming or which is dissolving.

5. The method as claimed in one or more of claims 1-4, wherein the suspendable particles are, for example, dye crystals, metal soles, silica particles, magnetic particles, oil drops, lipid particles, dextran, protein aggregates or, preferably, latex particles.

6. The method as claimed in one or more of claims 1-5, wherein the surface of the particles is modified further after the particles have been prepared and/or the particles are coated by one or more covalently or adsorptively bound layers or shells, e.g. composed of proteins, carbohydrates, lipophilic substances, biopolymers, organic polymers or mixtures thereof.

7. The method as claimed in one or more of claims 1-6, wherein photosensitizers and chemiluminescent compounds are employed as substances, and the singlet oxygen which is generated by the photosensitizer is able to activate the chemiluminescent compounds thereby causing them to emit light.

8. The method as claimed in one or more of claims 1-6, wherein photosensitizers and fluorescent compounds are employed as substances and the singlet oxygen which is generated by the photosensitizer is able to activate the fluorescent compound, thereby causing it to emit light, or to suppress the emission of light in a quenching process.

9. The method as claimed in one or more of claims 1-8, wherein acetone, benzophenone, 9-thioxanthone, eosin, 9,10-dibromoanthracene, chlorophyll, buckminsterfullerene, methylene blue, Rose Bengal, porphyrins, phthalocyanines and/or their derivatives are employed as photosensitizers.

10. The method as claimed in one or more of claims 1-9, wherein olefins, 9-alkylidenexanthans, 9-alkylidene-N-alkylacridans, enol ethers, enamines, arylvinyl ethers, dioxenes, arylimidazoles and/or lucigenin are employed as chemiluminescent compounds.

11. The method as claimed in one or more of claims 1-10, wherein chemiluminescent compounds are brought into contact with fluorophores which are able to be stimulated by the activated chemiluminescent compounds and emit light of higher wavelengths.

12. The method as claimed in one or more of claims 1-11, wherein fluorescent compounds are used which, as the result of reaction with singlet oxygen, are subject to a photooxidation (photobleaching), for example 1,3-diphenylisobenzofuran, or which react with singlet oxygen, as photoactive precursors, to give fluorophores, for example oxenumbelliferyl ethers or umbelliferyl selenides.

13. The method as claimed in one or more of claims 1-12 which can be used for detecting a genetically determined and/or acquired deficiency of factors of the blood coagulation system and fibrinolysis system, genetic defects of thrombocytes, hemostasis disturbances which are due to diseases or due to therapeutic agents which have been administered, and/or genetic and/or acquired defects of the complement system.

14. The method as claimed in one or more of claims 1-13, wherein the time required for forming a fibrin clot is determined.

15. The method as claimed in one or more of claims 1-14, wherein clot formation is induced in a plasma or at least in a medium containing fibrinogen and/or blood platelets by adding one or more substances which induce(s) coagulation.

16. The method as claimed in one or more of claims 1-15, wherein the activated partial thromboplastin time (APTT), the thromboplastin time (PT), the protein C activation time (PCAT), the Russell's viper venom time (RVVT) or the thrombin time is determined.

17. The method as claimed in one or more of claims 1-16, wherein, in order to determine the factors of the fibrinolysis system, the sample to be measured can be substituted with the factors which are required for clot formation, for example by means of mixing with a deficient plasma or adding purified factors such as uPA, tPA, α₂-antiplasmin, plasminogen, plasminogen activator inhibitor 1 and/or factor XII, in particular fibrinogen.

18. The method as claimed in one or more of claims 1-17, wherein a spontaneous lysis is induced or a lysis is induced after adding activators, such as factor XIIa, tPA, uPA or streptokinase, or directly after adding plasmin.

19. The method as claimed in one or more of claims 1-18, wherein the time at which the lysis of a fibrin clot begins is determined by means of a signal increase.

20. The method as claimed in one or more of claims 1-19, wherein the activity of the blood platelets is determined by way of their ability to aggregate.

21. The method as claimed in one or more of claims 1-20, wherein blood platelet aggregation can be induced by substances such as adenosine diphosphate, collagen, thrombin, serotonin or epinephrin, ionophores, complement or streptolysin.

22. The method as claimed in one or more of claims 1-21, wherein the concentration or the activity of pharmaceuticals is determined.

23. The method as claimed in claim 22, which is used for therapeutic drug-level monitoring.

24. The use of a diagnostic agent which comprises one or more substances which, as a consequence of blood platelet aggregation, clot formation and/or clot dissolution are brought to a distance from each other which permits or prevents a measurable interaction, in particular an energy transfer, between the substances, wherein at least one of the substances is able to bind nonspecifically, e.g. by means of hydrophobic or electrostatic interaction, directly or indirectly, to components of a blood clot which is forming or which is dissolving, and one or more of the substances are bound covalently, by way of a specific interaction and/or adsorptively to suspendable particles, and/or are incorporated into these particles or themselves constitute suspendable particles or a part thereof, for carrying out the method as claimed in one or more of claims 1-23.

25. The use of a diagnostic agent as claimed in claim 24 for detecting an acquired or inherited hemostasis disturbance.

## Revendications

1. Procédé pour la détection d'un trouble hémostatique, dans lequel, par suite d'une agrégation plaquettaire, de la formation d'un caillot et/ou de la désintégration d'un caillot, des substances sont mises entre elles à une distance qui permet ou empêche une interaction, en particulier un transfert d'énergie, entre les substances, et on mesure le degré de l'interaction, **caractérisé en ce qu'**une ou plusieurs de ces substances sont liées par covalence, par interaction spécifique et/ou par adsorption à des particules aptes à être mises en suspension et/ou sont incorporées dans celles-ci ou représentent elles-mêmes des particules aptes à être mises en suspension ou constituent une partie de celles-ci.

2. Procédé selon la revendication 1, **caractérisé en ce que** le transfert d'énergie s'effectue au moyen de molécules à courte durée de vie, par exemple l'oxygène singulet, un rayonnement à faible portée, par exemple le rayonnement β radioactif, et/ou par transfert d'énergie selon Förster.

3. Procédé selon la revendication 1 ou 2, **caractérisé en ce que** l'activité de substances est accentuée ou inhibée par d'autres substances et cela conduit à une modification de signal mesurable, comme par exemple la modification de l'intensité ou de la polarisation de la lumière émise, l'inhibition ou l'augmentation d'activités enzymatiques ou/et la modification des propriétés de fluorescence.

4. Procédé selon une ou plusieurs des revendications 1 à 3, **caractérisé en ce qu'**au moins l'une des substances peut se lier directement ou indirectement de façon non spécifique, par exemple par interaction électrostatique ou hydrophobe, à des composants d'un caillot de sang se formant ou se désintégrant.

5. Procédé selon une ou plusieurs des revendications 1 à 4, **caractérisé en ce que** les particules aptes à être mises en suspension consistent par exemple en des cristaux de colorants, des sols métalliques, des particules de silice, des particules magnétiques, des gouttes d'huile, des particules lipidiques, du dextrane, des agrégats de protéines ou de préférence des particules de latex.

6. Procédé selon une ou plusieurs des revendications 1 à 5, **caractérisé en ce que** la surface des particules est encore modifiée après leur préparation et/ou les particules sont enrobées d'une ou plusieurs couches ou enveloppes liées par covalence ou adsorption - par exemple à base de protéines, de glucides, de substances lipophiles, de biopolymères, de polymères organiques ou de mélanges de ceux-ci.

7. Procédé selon une ou plusieurs des revendications 1 à 6, **caractérisé en ce qu'**on utilise comme substances des photosensibilisateurs et des composés chimiluminescents et l'oxygène singulet engendré par le photosensibilisateur peut activer les composés chimiluminescents pour l'émission de lumière.

8. Procédé selon une ou plusieurs des revendications 1 à 6, **caractérisé en ce qu'**on utilise comme substances des photosensibilisateurs et des composés fluorescents et l'oxygène singulet engendré par le photosensibilisateur peut activer le composé fluorescent pour l'émission de lumière ou réprimer l'émission de lumière dans un processus d'extinction.

9. Procédé selon une ou plusieurs des revendications 1 à 8, **caractérisé en ce qu'**on utilise comme photosensibilisateur l'acétone, la benzophénone, des 9-thioxanthones, l'éosine, le 9,10-dibromoanthracène, la chlorophylle, des buckminsterfullerènes, le bleu de méthylène, le rose Bengale, des porphyrines, des phtalocyanines et/ou leurs dérivés.

10. Procédé selon une ou plusieurs des revendications 1 à 9, **caractérisé en ce qu'**on utilise comme composés chimiluminescents des oléfines, des 9-alkylidènexanthanes, des 9-alkylidène-N-alkylacridanes, des énoléthers, des énamines, des éthers arylvinyliques, des dioxènes, des arylimidazoles et/ou la lucigénine.

11. Procédé selon une ou plusieurs des revendications 1 à 10, **caractérisé en ce que** les composés chimiluminescents sont mis en contact avec des fluorophores qui peuvent être excités par les composés chimiluminescents activés et émettent de la lumière à plus grandes longueurs d'onde.

12. Procédé selon une ou plusieurs des revendications 1 à 11, **caractérisé en ce qu'**on utilise des composés fluorescents qui subissent une photo-oxydation *(photobleaching)* par réaction avec de l'oxygène singulet, comme par exemple le 1,3-diphénylisobenzofuranne, ou réagissent avec l'oxygène singulet en tant que précurseurs photoactifs pour donner des fluorophores, comme par exemple des oxénombelliféryléthers ou des séléniures d'ombelliféryle.

13. Procédé selon une ou plusieurs des revendications 1 à 12, utilisable pour la détection d'une déficience acquise et/ou d'origine génétique en facteurs du système de fibrinolyse et coagulation du sang, d'anomalies génétiques des thrombocytes, de troubles hémostatiques dus à des agents thérapeutiques administrés et/ou d'anomalies génétiques et/ou acquises du système du complément.

14. Procédé selon une ou plusieurs des revendications 1 à 13, **caractérisé en ce qu'**on détermine le temps jusqu'à la formation d'un caillot de fibrine.

15. Procédé selon une ou plusieurs des revendications 1 à 14, **caractérisé en ce qu'**on déclenche la formation d'un caillot dans un plasma ou au moins dans un milieu contenant du fibrinogène et/ou des plaquettes sanguines, par l'addition d'une ou plusieurs substances déclenchant la coagulation.

16. Procédé selon une ou plusieurs des revendications 1 à 15, **caractérisé en ce qu'**on détermine la temps partiel de thromboplastine activé (APTT), le temps de thromboplastine (PT), le temps d'activation de la protéine C (PCAT), le temps de venin de vipère de Russell (RWT) ou le temps de thrombine.

17. Procédé selon une ou plusieurs des revendications 1 à 16, **caractérisé en ce que**, pour la détermination des facteurs du système de fibrinolyse, on peut remplacer l'échantillon à doser par les facteurs requis pour la formation du caillot, comme par exemple par mélange avec un plasma déficient ou par l'addition de facteurs purifiés, comme par exemple l'uPA, le tPA, l'antiplasmine α₂, le plasminogène, l'inhibiteur d'activateur du plasminogène 1 et/ou le facteur XII, en particulier de fibrinogène.

18. Procédé selon une ou plusieurs des revendications 1 à 17, **caractérisé en ce qu'**on déclenche une lyse spontanée ou une lyse après addition d'activateurs, comme par exemple le facteur XIIa, le tPA, l'uPA, la streptokinase ou directement après addition de plasmine.

19. Procédé selon une ou plusieurs des revendications 1 à 18, **caractérisé en ce qu'**on détermine l'instant du début de la lyse d'un caillot de fibrine par une augmentation de signal.

20. Procédé selon une ou plusieurs des revendications 1 à 19, **caractérisé en ce qu'**on détermine l'activité des plaquettes par leur aptitude à l'agrégation.

21. Procédé selon une ou plusieurs des revendications 1 à 20, **caractérisé en ce que** l'agrégation des plaquettes peut être déclenchée par des substances telles que l'adénosine-diphosphate, le collagène, la thrombine, la sérotonine ou l'épinéphrine, des ionophores, le complément ou la streptolysine.

22. Procédé selon une ou plusieurs des revendications 1 à 21, **caractérisé en ce qu'**on détermine la concentration ou l'efficacité de médicaments.

23. Procédé selon la revendication 22, **caractérisé en ce qu'**on l'utilise pour la surveillance thérapeutique de taux de médicaments.

24. Utilisation d'un agent de diagnostic contenant une ou plusieurs substances qui, par suite d'une agrégation plaquettaire, de la formation d'un caillot et/ou de la désintégration d'un caillot, sont mises entre elles à une distance qui permet ou empêche une interaction, en particulier un transfert d'énergie, entre les substances, au moins une des substances pouvant se lier directement ou indirectement de façon non spécifique, par exemple par interaction électrostatique ou hydrophobe, à des composants d'un caillot de sang se formant ou se désintégrant, et une ou plusieurs des substances étant liée par covalence, par interaction spécifique et/ou par adsorption à des particules aptes à être mises en suspension et/ou étant incorporées dans celles-ci ou représentant elles-mêmes des particules aptes à être mises en suspension ou constituant une partie de celles-ci, pour la mise en oeuvre du procédé selon une ou plusieurs des revendications 1 à 23.

25. Utilisation d'un agent de diagnostic selon la revendication 24, pour la détection d'un trouble hémostatique acquis ou héréditaire.
